# EUROPEAN PATENT APPLICATION

(11) **EP 4 674 842 A1**
(43) Date of publication of application: **07.01.2026**
(21) Application number: 24763699.6
(22) Date of filing: 20.02.2024
(51) Int. Cl.: C07D 251/24, C07B 61/00

(54) **TRIAZINE COMPOUND AND METHOD FOR PRODUCING SAME**

(30) Priority: 02.03.2023 JP 2023031771
(71) Applicant: SHIN-ETSU CHEMICAL CO., LTD., Tokyo 1000005 (JP)
(72) Inventor: ISHIZAKI Shinichi, Annaka-shi, Gunma 379-0224 (JP); YAMAKI Masahiro, Annaka-shi, Gunma 379-0224 (JP); SUGO Michihiro, Annaka-shi, Gunma 379-0224 (JP)
(74) Representative: Ter Meer Steinmeister & Partner
(86) International application number: PCT/JP2024/005901
(87) International publication number: WO 2024/181218

(57) **Abstract**

Provided is a triazine compound represented by formula (1). 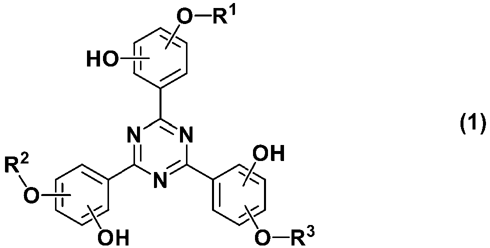 **[In** the formula, each of R¹-R³ is independently a hydrogen atom, an alkyl group, or a group represented by formula (2) or formula (3), and at least one thereof is a group represented by formula (3). 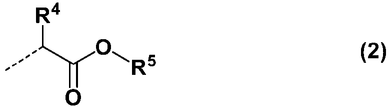 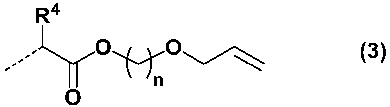 (In the formulae: each R⁴ is independently a hydrogen atom or a C1-C7 alkyl group; R⁵ is a C1-C12 alkyl group; and n is an integer of 1-5.)]

## Description

### TECHNICAL FIELD

This invention relates to a triazine compound and a method for preparing the same.

### BACKGROUND ART

In the prior art pertaining to triazine compounds terminated with an unsaturated group, a compound having the following formula (A) is known (see Patent Document 1). However, a triazine compound terminated with an unsaturated bond via a substituent derived from a glycol is not known.

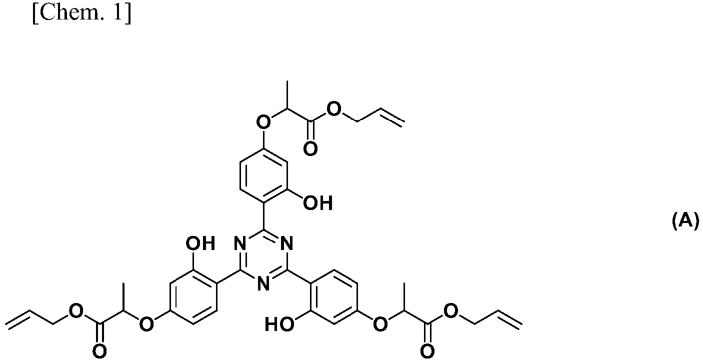

### PRIOR ART DOCUMENTS

### PATENT DOCUMENT

Patent Document 1: WO 01/062821

### SUMMARY OF INVENTION

### TECHNICAL PROBLEM

An object of the invention, which has been made under the above-mentioned circumstances, is to provide a novel triazine compound terminated with an unsaturated bond.

### SOLUTION TO PROBLEM

Making extensive investigations to attain the above object, the inventors have found that a triazine compound terminated with an unsaturated bond is obtained by reacting a triazine compound having the formula (4): 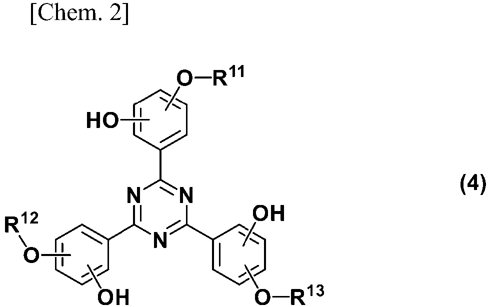 wherein R¹¹ to R¹³ are each independently hydrogen, an alkyl group, or a group having the formula (2), at least one of R¹¹ to R¹³ being a group having formula (2), 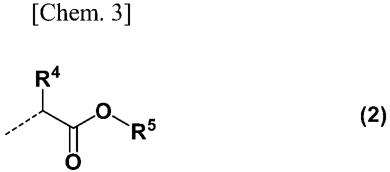 wherein R⁴ is each independently hydrogen or a C₁-C₇ alkyl group, and R⁵ is a C₁-C₁₂ alkyl group, with an allyl ether compound having the formula (5): 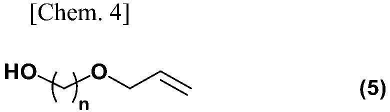 wherein n is an integer of 1 to 5. The invention is predicated on this finding.

The invention provides a triazine compound and a method for preparing the same, as defined below.
1. A triazine compound having the formula (1): 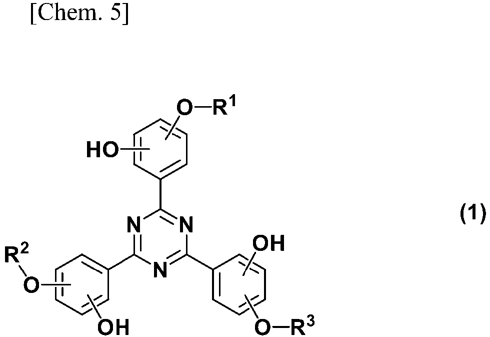 wherein R¹ to R³ are each independently hydrogen, an alkyl group, a group having the formula (2) or a group having the formula (3), at least one of R¹ to R³ being a group having formula (3), 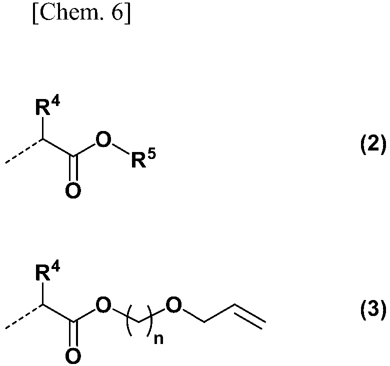 wherein R⁴ is each independently hydrogen or a C₁-C₇ alkyl group, R⁵ is a C₁-C₁₂ alkyl group, and n is an integer of 1 to 5.
2. The triazine compound of 1 wherein R⁴ is each independently hydrogen or methyl.
3. The triazine compound of 1 or 2 wherein R⁵ is methyl, ethyl, propyl or 2-ethylhexyl.
4. The triazine compound of any one of 1 to 3 wherein n is 2 or 3.
5. A method for preparing a triazine compound having the formula (1), comprising the step of reacting a triazine compound having the formula (4) with an allyl ether compound having the formula (5), 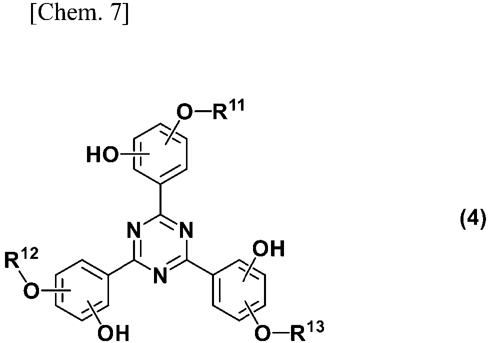 wherein R¹¹ to R¹³ are each independently hydrogen, an alkyl group, or a group having the formula (2), at least one of R¹¹ to R¹³ being a group having formula (2), 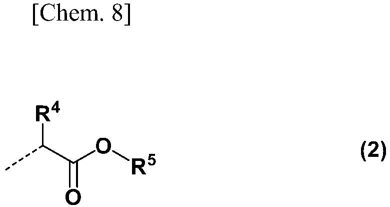 wherein R⁴ is hydrogen or a C₁-C₇ alkyl group, and R⁵ is a C₁-C₁₂ alkyl group, 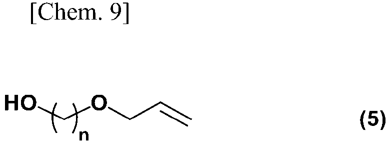 wherein n is an integer of 1 to 5, 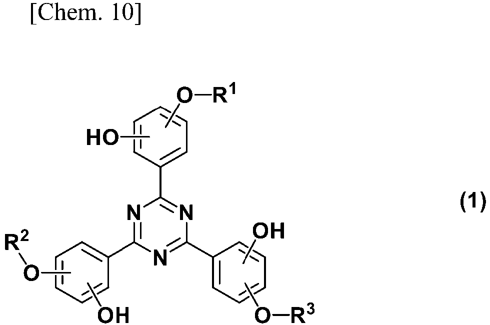 wherein R¹ to R³ are each independently hydrogen, an alkyl group, a group having the formula (2) or a group having the formula (3), at least one of R¹ to R³ being a group having formula (3), 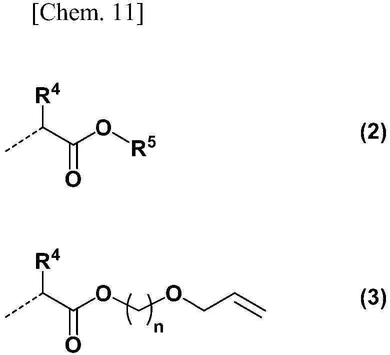 wherein R⁴ is each independently hydrogen or a C₁-C₇ alkyl group, R⁵ is a C₁-C₁₂ alkyl group, and n is an integer of 1 to 5.

### ADVANTAGEOUS EFFECTS OF INVENTION

According to the invention, a triazine compound terminated with an unsaturated bond can be prepared. This triazine compound is expected useful as a UV absorber. When the triazine compound is incorporated into a polymer by reacting the terminal unsaturated bond, there is obtained a UV absorber of polymer type, which is useful especially in the fabrication of semiconductor devices due to expectable improvements in chemical resistance and heat resistance.

### DESCRIPTION OF EMBODIMENTS

The invention provides a triazine compound terminated with an unsaturated bond, having the formula (1).

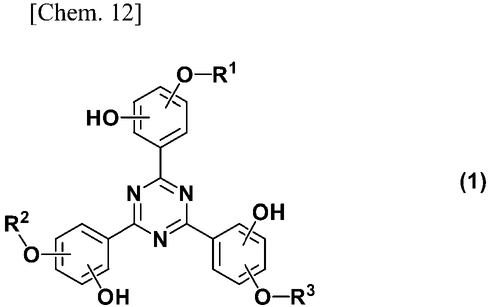

In formula (1), R¹ to R³ are each independently hydrogen, an alkyl group, a group having the formula (2) or a group having the formula (3), at least one of R¹ to R³ being a group having formula (3).

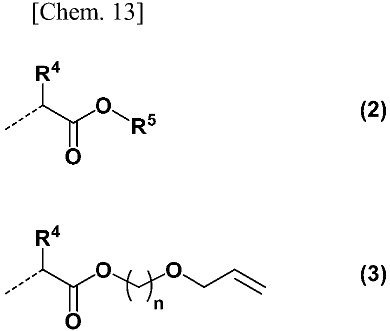

In formulae (2) and (3), R⁴ is each independently hydrogen or a C₁-C₇ alkyl group, R⁵ is a C₁-C₁₂ alkyl group, and n is an integer of 1 to 5.

Examples of the alkyl group represented by R¹ to R³ include C₁-C₁₂ alkyl groups such as methyl, ethyl, propyl, isopropyl, butyl, isobutyl, tert-butyl, pentyl, neopentyl, hexyl, heptyl, octyl, 2-ethylhexyl, nonyl, decyl and dodecyl. Of the alkyl groups, methyl, ethyl and propyl are preferred.

Examples of the C₁-C₇ alkyl group represented by R⁴ include methyl, ethyl, propyl, isopropyl, butyl, isobutyl, tert-butyl, pentyl, neopentyl, hexyl, and heptyl. Inter alia, R⁴ is preferably hydrogen or methyl.

Examples of the C₁-C₁₂ alkyl group represented by R⁵ include methyl, ethyl, propyl, isopropyl, butyl, isobutyl, tert-butyl, pentyl, neopentyl, hexyl, heptyl, octyl, 2-ethylhexyl, nonyl, decyl and dodecyl. Inter alia, R⁵ is preferably methyl, ethyl, propyl or 2-ethylhexyl.

In formula (3), n is an integer of 1 to 5, preferably 2 to 4, more preferably 2 or 3.

Preferably, all R¹ to R³ are groups having formula (3).

Examples of the compound having formula (1) are shown below, but not limited thereto.

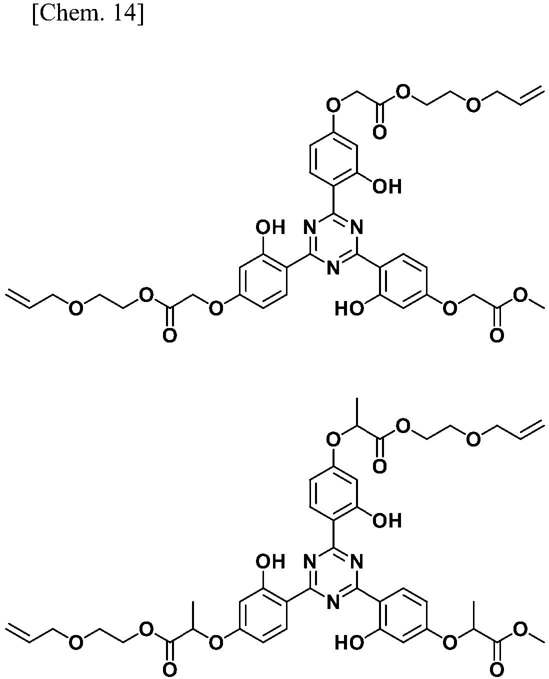

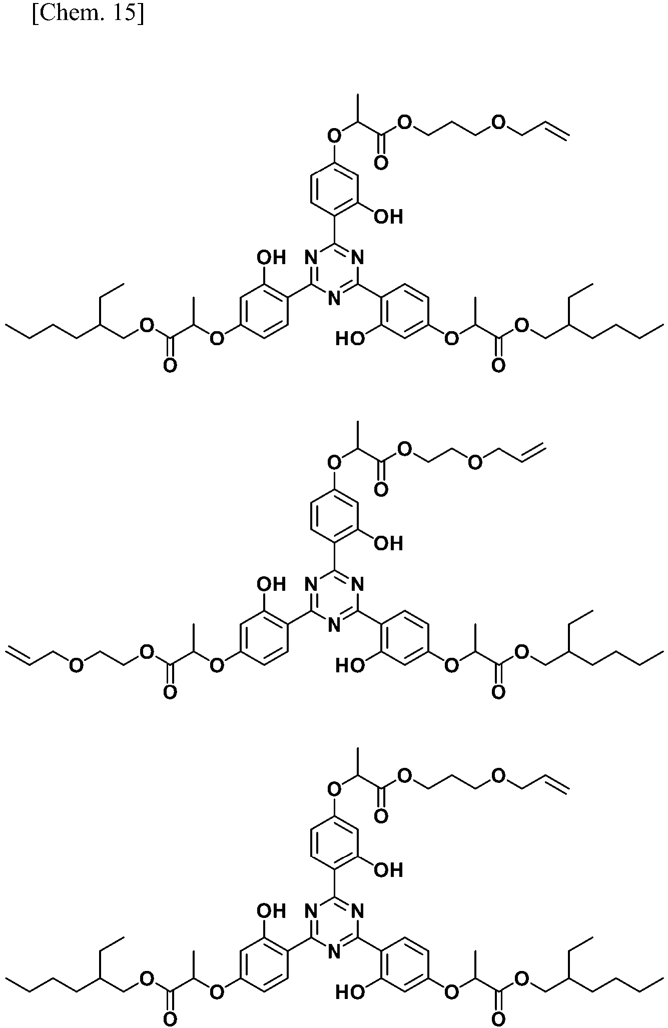

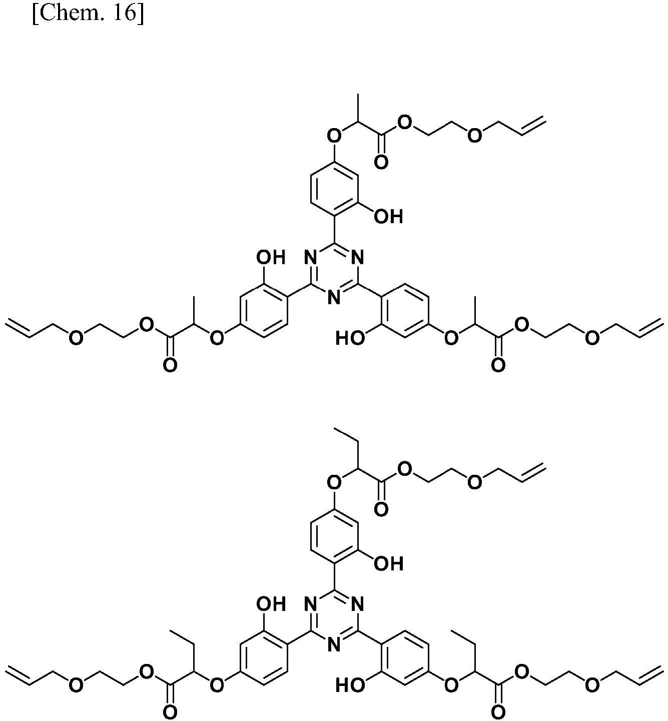

The compound having formula (1) is prepared by reacting a triazine compound having the formula (4): 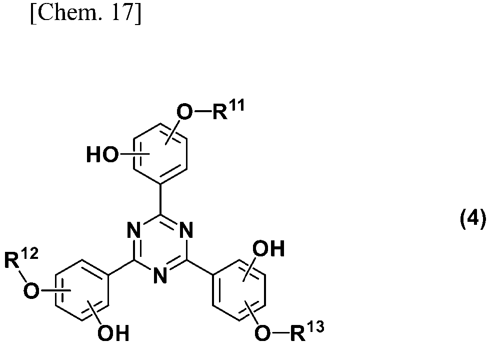 wherein R¹¹ to R¹³ are each independently hydrogen, an alkyl group, or a group having the formula (2), at least one of R¹¹ to R¹³ being a group having formula (2), 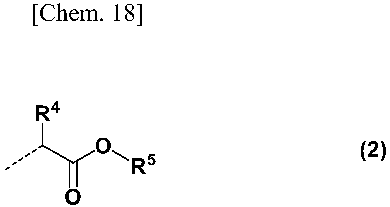 wherein R⁴ and R⁵ are as defined above, with an allyl ether compound having the formula (5): 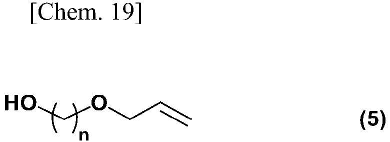 wherein n is an integer of 1 to 5.

Examples of the alkyl group represented by R¹¹ to R¹³ include C₁-C₁₂ alkyl groups such as methyl, ethyl, propyl, isopropyl, butyl, isobutyl, tert-butyl, pentyl, neopentyl, hexyl, heptyl, octyl, 2-ethylhexyl, nonyl, decyl and dodecyl. Of the alkyl groups, methyl, ethyl and propyl are preferred.

Examples of the compound having formula (4) are shown below, but not limited thereto.

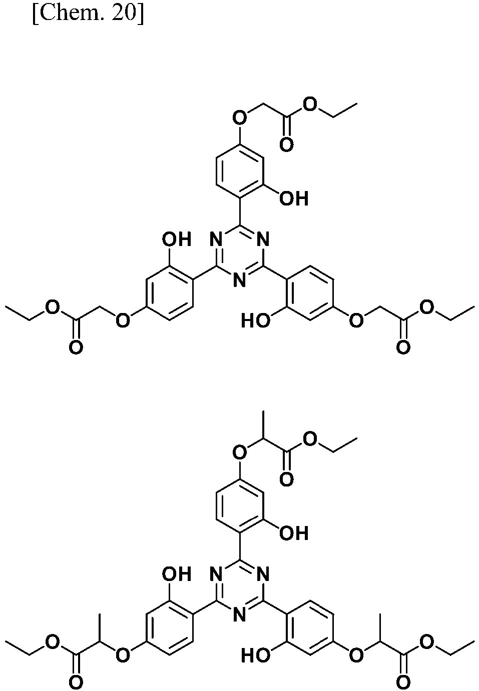

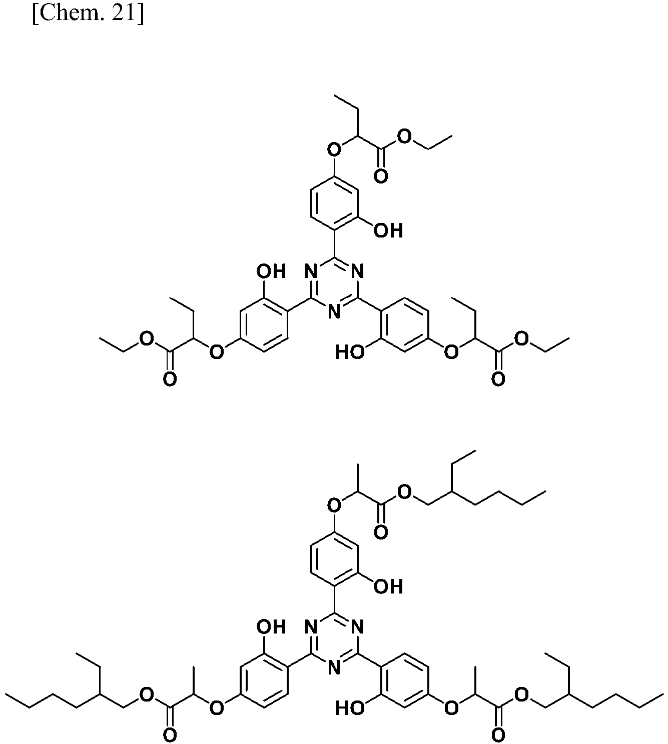

Examples of the allyl ether compound having formula (5) are shown below, but not limited thereto.

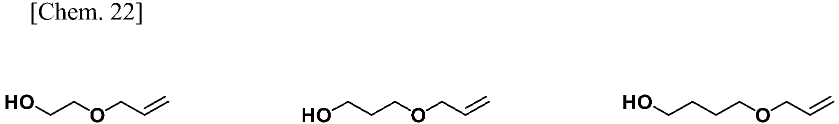

The amount of the allyl ether compound having formula (5) used is preferably 20 to 100 moles, more preferably 30 to 70 moles per mole of the compound having formula (4).

As to the solvent used in the reaction, it is possible that the allyl ether compound is added in large excess so that it may serve as the reaction solvent. It is optional to use another solvent. In this case, a solvent which is compatible with the compound of formula (4) and the allyl ether compound of formula (5) is preferred. Examples of suitable solvents include ethers such as diethyl ether, tetrahydrofuran and dioxane; aromatic hydrocarbons such as benzene, toluene and xylene; and halogenated hydrocarbons such as dichloroethane, chloroform and chlorobenzene.

An acid catalyst may be used in the reaction. Suitable acid catalysts include nitric acid, hydrochloric acid, sulfuric acid and fuming sulfuric acid. The amount of the acid catalyst used is preferably 0.1 to 1.0 equivalent, more preferably 0.1 to 0.2 equivalent per mole of the compound of formula (4).

The reaction may be carried out typically at 40 to 200°C, preferably 90 to 170°C although the reaction temperature is not particularly limited. The reaction time is about 2 to 30 hours, preferably 4 to 6 hours.

By washing the reaction product with water and removing the solvent or the like, the desired triazine compound is obtained. In the preferred procedure following the reaction, the acid catalyst and the like are removed by water washing or other means, and the excess of the allyl ether compound and solvent are distilled off by heating under reduced pressure, whereupon the desired triazine compound is obtained.

The triazine compound of the invention is useful as a UV absorber and effective in the fabrication of semiconductor devices.

### EXAMPLES

Examples of the invention are given below by way of illustration although the invention is not limited thereto. Notably, ¹H-NMR spectroscopy analysis was carried out using Bruker 400MHz.

### [Example 1]

A 1-L separable flask equipped with a nitrogen gas inlet tube, thermometer and Dimroth condenser was charged with 98 g (0.1 mol) of a compound having the formula (6). 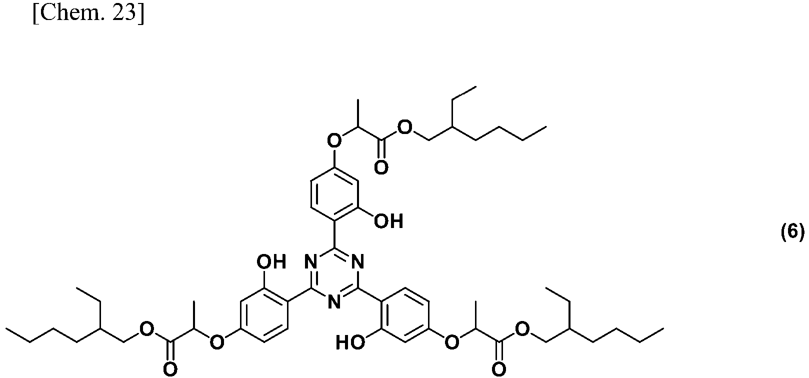 The compound was dissolved in 378 g (3.7 mol) of allyl glycol. To the solution, 1.00 g (0.010 mol) of sulfuric acid was slowly added. The solution was heated at 160°C, followed by 5 hours of reaction. At the end of reaction, 500 g of toluene and 1,000 g of deionized water were added to the reaction solution, which separated into the organic layer and the water layer. From the organic layer, allyl glycol and toluene were distilled off at 160°C under reduced pressure, obtaining 81 g of a reddish brown, high-viscosity liquid. On analysis by ¹H-NMR spectroscopy, the resulting compound was identified to be a triazine compound terminated with an unsaturated bond, having the formula (7). The data of ¹H-NMR spectroscopy are shown in Table 1.

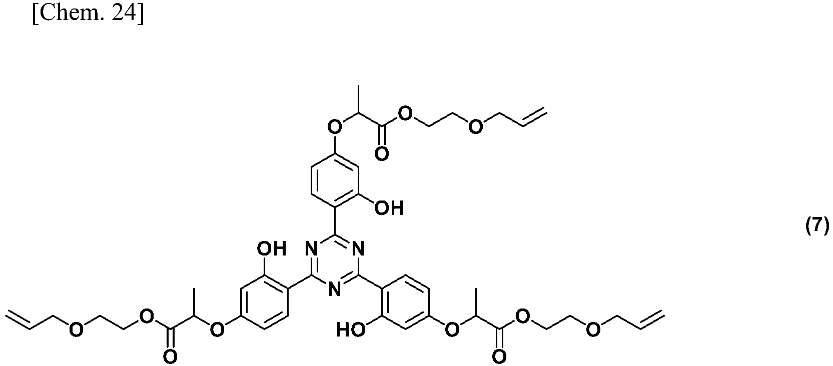

**[Table 1]**

| Peaks | δ, ppm | |
|---|---|---|
| f, f', f" | 1.66 - 1.68 | 9H |
| H, H', H" | 3.64 - 3.65 | 6H |
| i, i', I" | 3.99 - 4.00 | 6H |
| g, g', g" | 4.35 - 4.36 | 6H |
| e, e', e" | 4.84 - 4.87 | 3H |
| e, e', e" | 4.84 - 4.87 | 3H |
| k, l, k', l', k", l" | 5.16 - 5.28 | 6H |
| j, j', j" | 5.82 - 5.91 | 3H |
| c, d, c', d', c", d" | 6.42 - 6.54 | 6H |
| b, b', b" | 7.91 - 7.94 | 3H |
| a, a', a" | 13.19 - 13.35 | 3H |

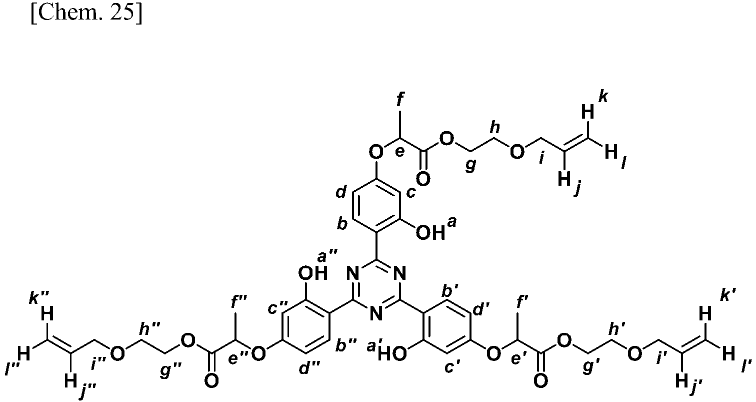

### [Example 2]

A 1-L separable flask equipped with a nitrogen gas inlet tube, thermometer and Dimroth condenser was charged with 75 g (0.1 mol) of a compound having the formula (8). 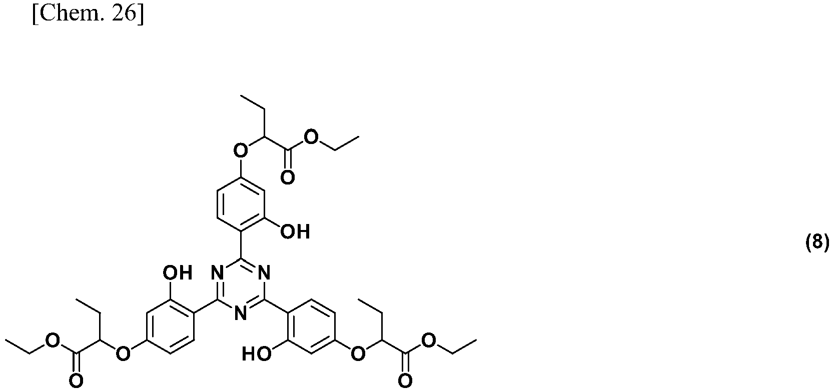 The compound was dissolved in 378 g (3.7 mol) of allyl glycol, to which 1.00 g (0.010 mol) of sulfuric acid was slowly added. The solution was heated at 160°C, followed by 5 hours of reaction. At the end of reaction, 500 g of toluene and 1,000 g of deionized water were added to the reaction solution, which separated into the organic layer and the water layer. From the organic layer, allyl glycol and toluene were distilled off at 160°C under reduced pressure, obtaining 82 g of a reddish brown, high-viscosity liquid. On analysis by ¹H-NMR spectroscopy, the resulting compound was identified to be a triazine compound terminated with an unsaturated bond, having the formula (9).

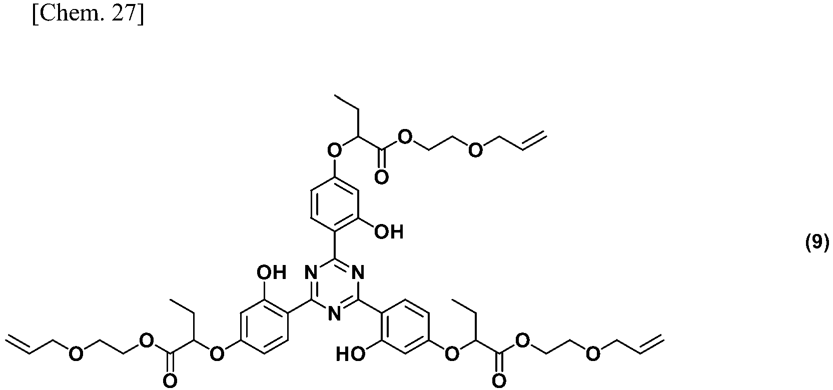

### [Example 3]

A 1-L separable flask equipped with a nitrogen gas inlet tube, thermometer and Dimroth condenser was charged with 66 g (0.1 mol) of a compound having the formula (10). 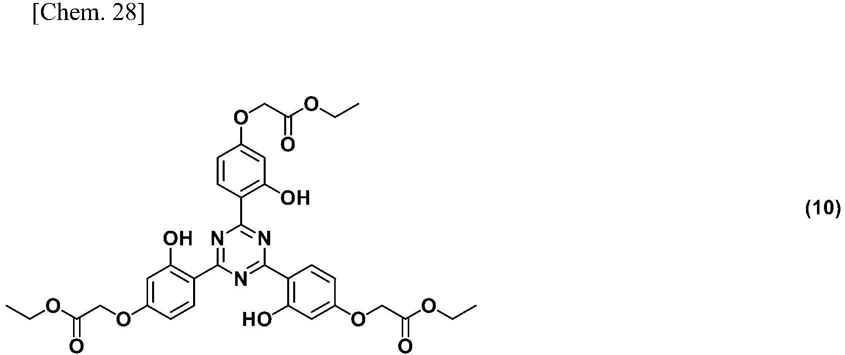 The compound was dissolved in 378 g (3.7 mol) of allyl glycol, to which 1.00 g (0.010 mol) of sulfuric acid was slowly added. The solution was heated at 160°C, followed by 5 hours of reaction. At the end of reaction, 500 g of toluene and 1,000 g of deionized water were added to the reaction solution, which separated into the organic layer and the water layer. From the organic layer, allyl glycol and toluene were distilled off at 160°C under reduced pressure, obtaining 72 g of a reddish brown, high-viscosity liquid. On analysis by ¹H-NMR spectroscopy, the resulting compound was identified to be a triazine compound terminated with an unsaturated bond, having the formula (11).

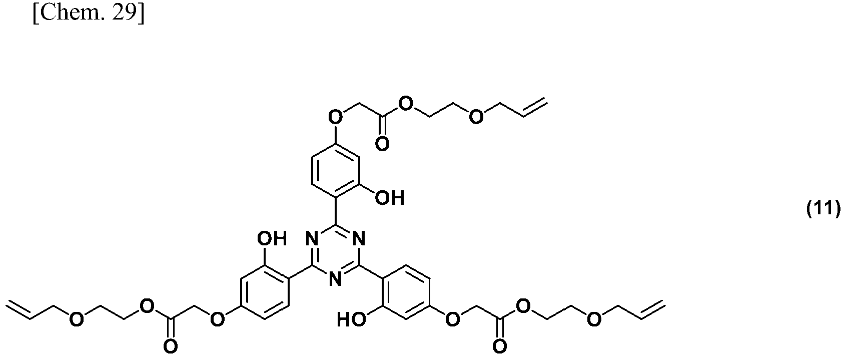

## Claims

1. A triazine compound having the formula (1): 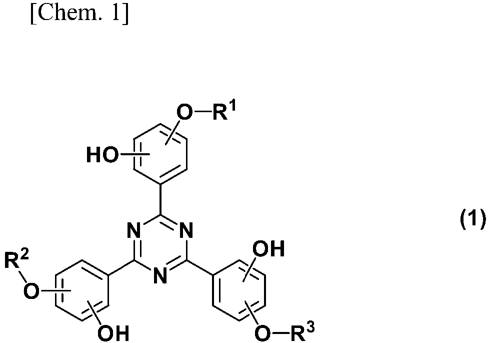 wherein R¹ to R³ are each independently hydrogen, an alkyl group, a group having the formula (2) or a group having the formula (3), at least one of R¹ to R³ being a group having formula (3), 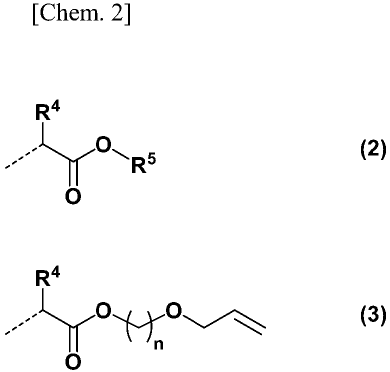 wherein R⁴ is each independently hydrogen or a C₁-C₇ alkyl group, R⁵ is a C₁-C₁₂ alkyl group, and n is an integer of 1 to 5.

2. The triazine compound of claim 1 wherein R⁴ is each independently hydrogen or methyl.

3. The triazine compound of claim 1 wherein R⁵ is methyl, ethyl, propyl or 2-ethylhexyl.

4. The triazine compound of any one of claims 1 to 3 wherein n is 2 or 3.

5. A method for preparing a triazine compound having the formula (1), comprising the step of reacting a triazine compound having the formula (4) with an allyl ether compound having the formula (5), 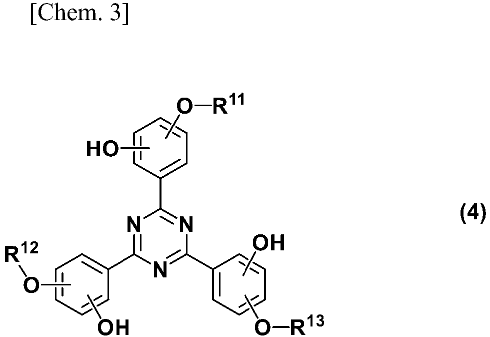 wherein R¹¹ to R¹³ are each independently hydrogen, an alkyl group, or a group having the formula (2), at least one of R¹¹ to R¹³ being a group having formula (2), 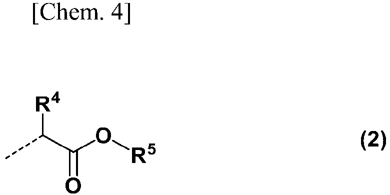 wherein R⁴ is hydrogen or a C₁-C₇ alkyl group, and R⁵ is a C₁-C₁₂ alkyl group, 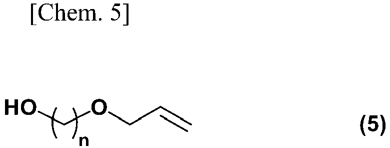 wherein n is an integer of 1 to 5, 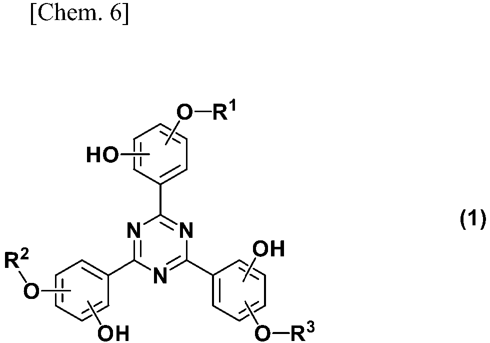 wherein R¹ to R³ are each independently hydrogen, an alkyl group, a group having the formula (2) or a group having the formula (3), at least one of R¹ to R³ being a group having formula (3), 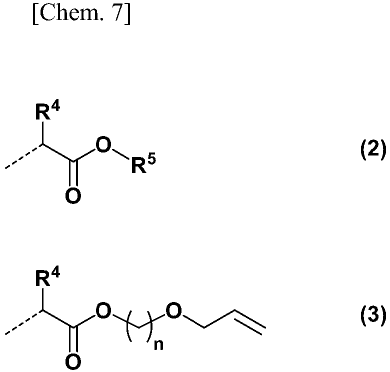 wherein R⁴ is each independently hydrogen or a C₁-C₇ alkyl group, R⁵ is a C₁-C₁₂ alkyl group, and n is an integer of 1 to 5.
